# EUROPEAN PATENT SPECIFICATION

(11) **EP 0 367 743 B1**
(45) Date of publication and mention of the grant of the patent: **01.06.1994**
(21) Application number: 89850373.5
(22) Date of filing: 30.10.1989
(51) Int. Cl.: C07C 31/20, C07C 69/28, C07C 29/38, C07C 67/44

(54) **Process for the preparation of branched 1,3-glycols and their monoesters**
Verfahren zur Herstellung von verzweigten 1,3-Glykolen und ihren Monoestern
Procédé de préparation de glycols-1,3 ramifiés et de leurs monoesters

(30) Priority: 02.11.1988 SE 8803978
(43) Date of publication of application: 09.05.1990
(73) Proprietor: NESTE OXO AKTIEBOLAG, 444 84 Stenungsund (SE)
(72) Inventor: Ankner, Kjell, S-43543 Mölnlycke (SE); Carlsson, Pia, S-161 30 Bromma (SE); Hopfinger, Alfred, S-12442 Bandhagen (SE); Rahkola, Hakan, S-44460 Stenungsund (SE); Sjöberg, Kjell, S-18274 Stocksund (SE); Söderlund, Monica, S-11651 Stockholm (SE)
(74) Representative: Inger, Lars Ulf Bosson

(56) References cited:
- EP-A- 0 186 076

## Description

The present invention relates to a process for the preparation of branched 1,3-glycols and their monoesters by means of a catalytic condensation reaction of aldehydes comprising a hydrogen atom in alfa position.

The summary reaction can be described using the following formulae: 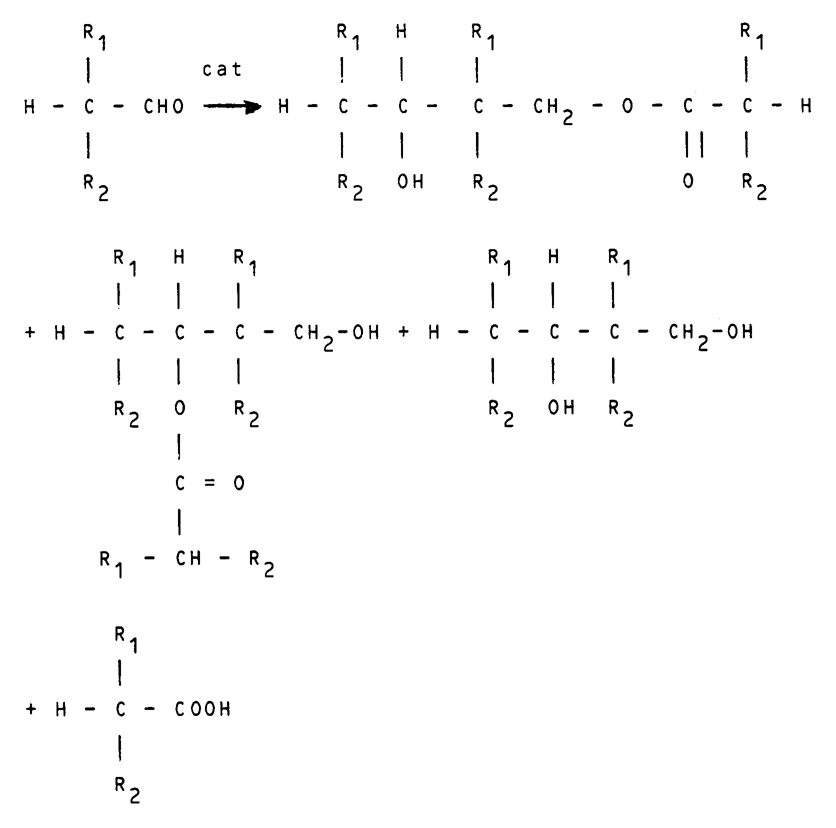
wherein R₁ and R₂ are the same or different and are each lower alkyl with 1 to 4 carbon atoms, preferably methyl. It is probable that some amounts of 1,3-glycols are formed directly by means of an aldolization of the starting aldehyde by the following reaction according to Canizarro:

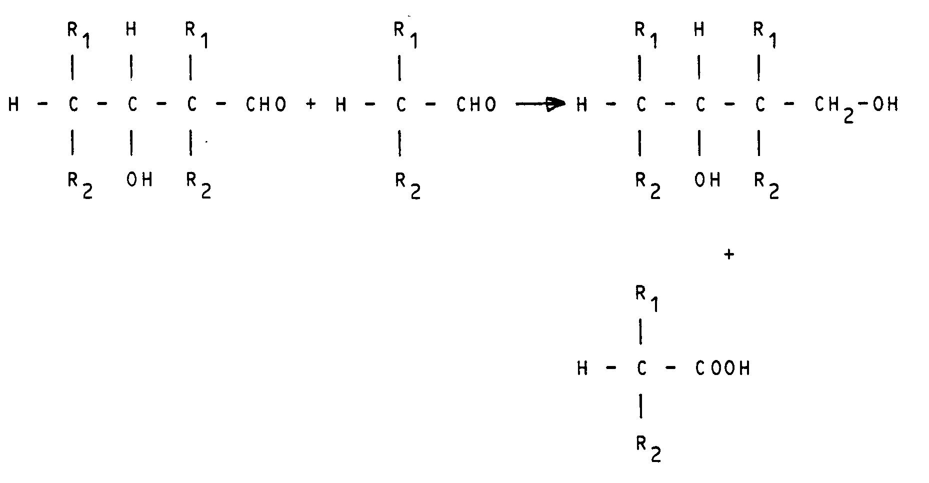

### Background of the invention

The above described reactions are known since 50 years - cf. for example DE-C-646,482 from 1937 - but different technical problems in large scale production have raised and requested improvements. Thus patent publications are published still today which relates to improvements of the preparation process. Another factor which has stimulated the interest for the problem is the growing field of use both of the glycol as such, as of its monoester. The industrially largest raw material among the aldehydes which have a hydrogen atom in alfa position is the isobutyric aldehyde. It is formed in considerable quantities at the hydroformylation of propene, the so called OXO-process, as a by-product to n-butyric aldehyde.

As condensation catalysts alkaline compounds has hitherto exclusively been used, compounds which are most often readily dissolved in water, such as sodium hydroxide, and potassium hydroxide. Hydroxides of metals belonging to group 2A of the periodic system show a satisfying catalytic activity. As the water is only slightly soluble in the reaction medium and inorganic hydroxides are practically insoluble in the organic phase the condensation reaction continues in a two phase system and when using the hardly soluble hydroxides of the group 2A in a three-phases system. Most patents of a later date describes different methods with regard to optimization of the difficulty controlled condensation reaction, but they are all connected with different drawbacks.

US-A-3,718,689 puts forward demands both of the purity of the isobutyric aldehyde - less than 0.5% of water and less than 0.5% organic acids. One also points at the necessity of powerful stirring in order to obtain a stable emulsion in a two phase system of concentrated hydroxide-aldehyde. The reaction time is considerably long and a satisfying yield is obtained first after about 2 hrs. Tables show that the reproducibility of the process is very low. Thus in accordance with Table II of said patent specification experiments 4, 5, and 6 have been carried out at apparently identical conditions, but still quite different yields of monoester were obtained, viz. 61%, 45%, and 20%, respectively.

A similar description of an alkali catalysed process is found in US-A-3,291,821 whereby a powerful stirring, and an intense circulation are said to be necessary. Although a powerful stirring is present the yield of the batchwise process is still low, only about 13%.

According to DE-A1-3,024,496 a stream of aldehyde and a stream of NaOH solution are introduced in parallel in a batchwise reactor. In this way one controls the intense evolution of heat and avoids the very rapid start of the reaction. This is particularly important if the reactor already from the beginning is completely filled by the reactants. One serious drawback which this process leads to is that it will become impossible to use it as a continous process - which has also been pointed at in said patent specification.

In US-A-3,703,541 the use of phenolic salts as reaction catalysts is described and according to US-A-3,091,632 sodium alkanoates are used. Both these types of catalysts increases to some extent the yield and the selectivity, but on the other hand they put hard requirements as to the purity of the raw material and particular requirements as to the absence of water in the reaction environment, which leads to that the processes are hardly attractive from an industrial point of view.

DE-A1-2,820,518 discloses a catalytic system consisting of alkali earth metal hydroxides and carboxylic acids and their salts, respectively. A serious drawback using this process is that one has to precipitate the alkali earth metal catalyst using gaseous carbon dioxide after the final reaction. A troublesome separation of the fine grained precipitate will then become necessary.

DE-A1-3,403,696 discloses a two step process consisting of a condensation reaction followed by a hydrogenation of raw reaction mixture at 120°C and a pressure of 100 bars. It is not probable that such a process can be competitive against other methods described above, which uses conventional reactors provided with stirrers and tube reactors, respectively, only.

According to DE-A1-3,447,029 one obtains a good contact between water and organic phase by using a tube reactor containing filler bodies - something that is known per se since long.

US-A-4,225,726 discloses a condensation reaction where tin, and tinoxide respectively are used as catalysts. The advantage is that these catalysts make it possible to carry out synthesis where aldehydes containing both one and two hydrogen atoms in alfa position are used as a raw material. The yield of the condensation product containing only one hydrogen atom in alfa position which is concerned in the present invention is, when using isobutyric aldehyde, only 20%.

### Description of the present invention

All the above described difficulties can easily be reduced by using the present invention for the preparation of branched 1,3-dioles of the general formulae I 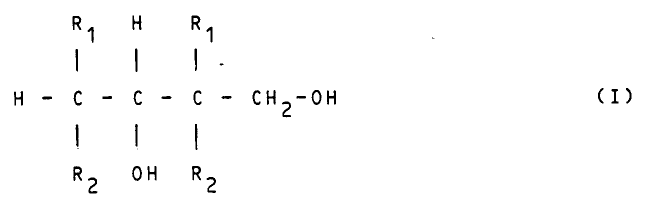
and/or its monoesters of the general formulae II 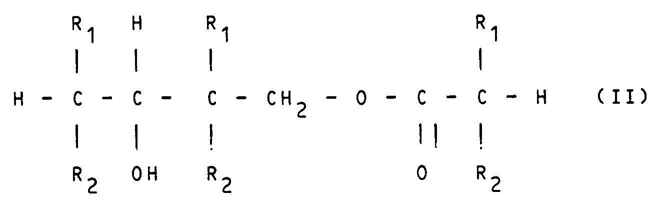
and/or the general formulae III 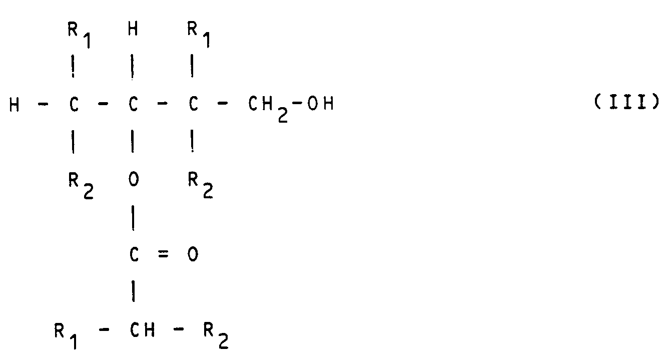
wherein R₁ and R₂ are the same or different and each denotes a lower alkyl having 1 to 4 carbon atoms, preferably methyl whereby the invention is characterized in that aldehydes comprising a hydrogen atom in alfa position are condensed in a multi phase system, which aldehydes are of the formulae IV 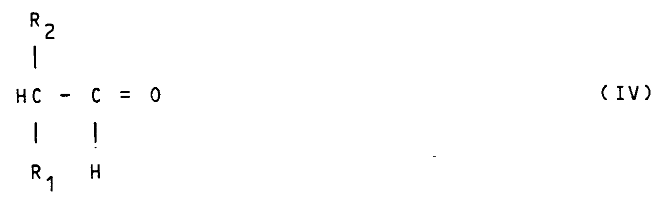
wherein R₁ and R₂ have the meaning given above, in the presence of a catalytic system consisting of a phase transferring catalyst and an alkaline substance.

The invention is based on the use of a catalytic system consisting of a phase transferring catalyst in the presence of an alkali. This gives a number of important technical advantages:
a) the reaction proceeds with a high speed;
b) one obtains a high yield of reaction products;
c) there is formed small amounts only of by-products;
d) the process can be controlled either to the formation of diol or monoester as main product or to the formation of an arbitrary mixture of these two products;
e) the catalyst can be recirculated;
f) it is enough using a moderate stirring of the reaction mixture;
g) the synthesis can be carried out either batchwise or continuously.

The phase transferring catalyst facilitates transport of hydroxy ions from the aqueous phase to the organic phase or to the phase border. As catalysts quartenary ammonium-, phosphonium-, and arsonium salts are most often used, as well as cyclic and straight aliphatic polyethers as well as certain chelate forming agents.

When an onium salt is being introduced into an alkaline aqueous solution the following ion exchange can take place 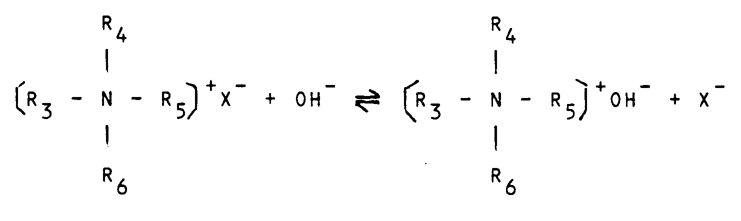
wherein R₃, R₄, R₅ and R₆ are hydrocarbon radicals and X⁻ is an anion.

The ion pair formed is extracted from the aqueous phase to the organic phase or border surface where the OH⁻ group catalyses the condensation reaction. Phase transferring catalysts of polyether structure behaves somewhat different in that they form a chelate compound with a metal cation, while the hydroxy group lies outside the chelate structure. The system polyether-metal cation-hydroxy ion formed is easily extracted to the organic phase.

There are three factors which decide the extractability of the OH⁻ group to the organic phase or the concentration at the phase border. Primarily: the concentration of the hydroxy anion in the aqueous phase - the more concentrated the aqueous solution is the greater the disposition of the ion pair to be transferred to the organic phase. Secondly: the hydrofobicity of the phase transfer catalyst - the more carbon atoms the onium salt contains, the higher will the extraction coefficient become. The co-operation between these parameters opens great optimizing possibilities with regard to reaction speed, selectivity, and design of the reactor.

Among different types of hydroxy ion formers hydroxides and oxides belonging to the first and second group A are most favourable, i.e. sodium hydroxide, potassium hydroxide, lithium hydroxide, calcium hydroxide, magnesium hydroxide, barium hydroxide. Preferably sodium hydroxide and potassium hydroxide are used in technical industrial processes. The concentration of hydroxy ions in an aqueous phase may vary within a broad range.

Even a 5% solution can give a good yield of the end product if the catalyst is hydrophobic enough. On the other hand one may use a 50% solution of alkaline substance whereby the reaction proceeds very rapidly but the end product may become yellow or brown coloured. The optimal concentration is in the range of 15 to 40%. The amount of hydroxide used also has a meaning as to which end product shall be obtained. The monoesters can suitably be produced using catalytic up to molar amounts of hydroxy ions, while the diol demands at least molar amount of hydroxy ions to be completely formed. In the presence of a phase transferring catalyst the hydrolysis of the monoester formed runs very fast and quantitatively.

Extraction of hydroxy ion from the aqueous phase to different types of organic phase using phase transfer catalysts has been investigated during the last years by a row of authors (Dehmlov, E.V., Dehmlov, S.S. - Phase Transfer Catalysis - II ed. - Verlag Chemie, p.33-41; Landini, D., Maja, A., Montanari, F., Israel Jour. of Chemistry, 26, (1985), p. 263-269; Gokel, G.W., Goli, D.M., Schulz, R.A., J. Org. Chem., 48, (1983), p. 2837-42; Dehmlov, E.V., Slopianka, M., Heider, J., Tetrahedron Lett. (1977), p. 2361). Their general conclusion is that the more hydrophobic the onium catalyst is the more effective it will transport the OH⁻ ion in the organic phase. The efficiency of the polyethers are depending on the chemical structure of the organic phase.

The experiments carried out and shown below show that extraction of hydroxy ion from aqueous phase to a mixture of isobutanal and its reaction products takes place in a satisfying way using onium catalysts containing at least 8 carbon atoms in their structure. The following cation structures showed to be particularly suitable: trioctyl ammonium; lauryl benzylammonium; methyl triheptylammonium; tetrabutylammonium; tetraisopropylammonium; tetraethylammonium; tetrapentylammonium; tetrahexylammonium; cetyltributylphosphonium; ethyl tributylphosphonium; tetrabutylphosphonium; benzyl triethylphosphonium. As anion to these quartenary cation structures sulphate, bromide, chloride acetate and other not acid and/or not too lipophilic anions be used. Very interesting results were obtained using straight chained polyethers. The polyglycolether having a mean molecular weight of between 200 and 400 turned out to be a very efficient phase transfer catalyst. Higher polymers of ethylene oxide are catalytically active, as well, but increasing viscosity of the reaction mixture makes their technical use more difficult.

The amount of the phase transfer catalyst can be varied within rather wide limits, between 0.02 to 10 mol-% calculated in relation to the amount of starting aldehyde. The optimal interval is between 0.25 to 4 mol-%. After finalized reaction the catalyst is separated depending on its chemical structure in different quantitative relationships in the aqueous and organic layers. It can also be separated from the two phases and be recirculated. Separation from the aqueous phase takes place using an extraction agent, where particularly lower esters and chlorinated hydrocarbons are suitable. Separation from the organic phase is most convenient done by means of distillation where one recovers the catalyst as a distillation residue. If the diol is being purified by recrystallisation the catalyst will end up in the mother liquid and can be easily separated as a residue at the regeneration of the solvent. The organic layer can most often be freed from catalyst by extraction using a weak alkaline aqueous solution.

The reaction temperature can vary within a range of between 40°C and 150°C. In order to reach the upper limit it is necessary to use a high over pressure. This is not profitable in practise as the reaction proceeds with a satisfying speed already at 60 to 90°C. This means that the working pressure of the process can be between 0,78 to 3,92 bar (0.8 to 4 atmospheres absolute pressure).

The catalytic synthesis method described facilitates the use of different types of reactors. One can thus carry out the reaction both batchwise and continuously. Short reaction times - particularly at the synthesis of monoesters - facilitates the use of tube reactors, as it is hereby not needed any particular intensive stirring. It is as simple as that to use through flowing tank reactors either in the form of single apparatuses or in the form of batteries of apparatuses of two or more units. A interesting solution of a tube reactor design is a combination of tube reactor and tank reactor. If one wants to produce mainly monoesters then one uses the tube reactor only where the staying time seldom exceeds 10 min. On the other hand if one should want to have the diol only or mainly then one introduces the stream of reactants from the tube reactor into a larger through flow tank reactor where a complete hydrolysis takes place. The process is most easily finished by stopping the stirring. It is then quite rapidly formed two separate phases and the reaction stops when the OH⁻ ions present in the organic layer have been used. A similar result is obtained by introducing an acid in the reaction environment, which neutralizes the free alkaline compounds, and by cooling down the reaction mixture, respectively.

If the process proceeds batchwise then it is convenient to first introduce the catalytic system, i.e. water, hydroxide/oxide, and catalyst into the reactor, heating this mixture to about 60°C and then introduce the aldehyde. In the presence of the phase transfer catalyst the reaction starts without any introductory period. The dose rate is determined in practise of the efficiency of a reflux cooler arranged in the system. After finished dosage the reaction mixture is stirred for some time. If it is a monoester which is produced and is the reaction product wanted the stirring time should not exceed a couple of minutes and a small amount of hydroxide ions should be used. If it is the diol which is the product wanted the reaction depends on molar amount of hydroxide ions. The stirring time may vary between 5 to 90 min. One may also add the alkaline solution and the aldehyde in parallel, but then, one should have introduced the total amount of phase transfer catalyst already from the beginning, dissolved or suspended in water into the reactor. One might also control the process course by using solvents, in particular aromatic hydrocarbons and higher alcohols. In the presence of solvents the reaction mixture will boil less intensive and the colour of the products will improve to some extent as well. The separation of the catalyst may also be made easier.

As have been disclosed in DE-A1-3,102,826 and DE-A1-3,024,496 it is advantageous to free the reaction mixture from the readily boiling products and to decompose the trimeric structures of the starting aldehyde by distillation in the presence of water, which is introduced after finished synthesis. In order to achieve a complete decomposition it might be necessary to use a temperature of up to 150°C and 10 bar super pressure, (Cf DE-A1-3,024,496, p.5). The same result can be obtained at considerably milder conditions if the distillation of the system of reaction mixture and water proceeds in the presence of phase transfer catalyst. Then it is enough using atmospheric pressure, and a slight over pressure, respectively, which is created under the influence of the flowing resistance. The temperature in the distillation head will thereby not exceed 80°C and the decomposition of the polymeric structures continues very fast. Most often the amount of catalyst present in the organic phase is enough after finished synthesis step. In certain cases it can, however, turn out necessary, to add a minor amount of fresh catalyst. There are two ways to add water to the system: either in one step to the distillation vessel or it is added continuously during the course of the distillation into the head of the column or directly into the vessel. The adding of hot water is preferred prior to adding cold water.

### Example 1.

In a flask provided with a ref lux cooler, a stirrer, a bottom valve, a dosing funnel, and a theremometer 50 g of o-xylene, 80 g of 25% NaOH aqueous solution, and 2.5 g of trioctylmethyl ammoniumchloride were added. The mixture was heated while stirring, to 60°C. At this temperature the addition of 144 g of isobutanal is started. The time for addition is 13 min and the temperature increases under moderate boiling to 77C. Then the temperature falls slowly to 60°C and is kept at this temperature to the end of the test. Under the course of the reaction samples are taken out to chromatographic analysis, shortly after completion of the addition, and 5, 10, 30, 60, and 180 min. after completion of the addition. Analytical data gave the following information: a) summary yield of ester and diol was not changed after stirring for 5 to 10 min, b) after this time hydrolysis of the monoester took place only, and it was finished after a maximum of 30 min., c) in the reaction mixture only minimal amounts of by-products were found.

After finished reaction the phases were separated. The organic phase was evaporated in vacuo at 60°C. The residue consisted of water clear liquid containing white crystals. It consisted of the monoester (2 isomers) and the diol in a molar ratio of 1.3:1. Summary yield of these products was 47%. The aqueous phase did not contain any free sodium hydroxide. After treatment with conc. hydrochloric acid an organic layer was formed consisting of isobutyric acid and a small amount of neutral compounds. Yield of crude isobutyric acid was 41 g.

### Example 2

In order to the influence of the phase transfer catalyst the experiment according to claim 1 was repeated but without any use of trioctylmethyl ammoniumchloride. During the addition of isobutanal no reaction took place. First after 25 min of stirring at 60°C the reaction was suddenly started and the temperature raised to 76°C. The stirring continued and samples were taken out in accordance with Ex. 1 . The reaction was practically finished after 10 min. but the summary yield of the reaction product was only 20%, of which 8% was diol.

### Example 3

The experiment according to Ex. 1 was repeated with the difference that methyl trioctylammoniumchloride (Aliquat 336, Henkel, DE) was used as phase transfer catalyst. The results obtained corresponded completely with those obtained in Ex. 1.

### Example 4

The experiment according to Ex. 1 was repeated with the difference that tetrabutylammoniumsulphate was used as phase transfer catalyst. The results obtained corresponded completely with those obtained in Ex. 1.

### Example 5

The object of this experiment was to obtain a major part as monoester. Hereby the experiment in accordance with Ex. 1 was repeated but after an addition time of 13 min. the stirring took place during 90 s only, whereupon the the stirring was stopped and the two phases were separated. The organic phase was distilled. The main product consisted of 12.3 mol-% of diol and 87.7 mol-% of monoester. The yield of the main product was 55.3 %.

### Example 6

In a flask provided with a bottom valve, reflux cooler, stirrer, addition funnel, and a thermometer there were introduced 40 g of a 20% NaOH solution and 16 g of polyethylenglycol having a mean molecular weight of 400. The contents of the flask was heated while stirring to 60°C. 144 g of isobutanal were added during 4.5 min. While adding the temperature raised to 77°C and the reaction mixture boiled. After finished addition the temperature was increased to 80°C and was kept at this level for 2 hrs. Sampies for analysis were taken out 1, 3, 8, 15, 30, and 60 min. after finished addition. The analysis showed that the reaction was completed after 8 min. After 2 hrs stirring the phases were separated and one obtained thereby 64.6 g aqueous phase and 123 g of organic phase. Loss as well as material for analysis were 12.4 g. The aqueous phase was acidified with an excess of hydrochloric acid and one obtained thereby 30.7 g of water insoluble layer consisting of isobutyric acid mixed with small amounts of neutral substances. The organic phase was distilled in the presence of water and catalyst. The residue consisted of a water clear liquid (94.2 g) consisting of the monoester, and diol, and a small amount of catalyst. By distillation in vacuo 81 g of the main product were obtained consisting of the monoester and diol in a molar ratio of 5:1. 10 g of the residue consisted of recovered catalyst mixed with the reaction products.

Distillation of the acidic phase separated from the water phase gave 20 g of a distillate and 10 g of a residue.

The distillation residues containing the catalyst were mixed together 5 g of fresh catalyst were added and the synthesis was repeated. The reaction course was the same as during the first, above described run.

### Example 7

The apparatus of a continuous process consisted of two dosing pumps of which one added isobutanal containing 2.8 % by weight of polyethylene glycol (M_{w} 400) and the other added 15% NaOH solution. The amount of NaOH as 100% was equal to 3.4 % by weight of the amount of isobutanal. The two solutions were pumped through heated glass tubes. where they were heated to 55°C and were then introduced into a stirring reactor consisting of a 100 ml flask provided with an inlet and an outlet tube, a thermometer, a reflux cooler, and a stirrer with its shaft through the reflux cooler. The average dwell time of the reaction mixture in the reactor was 1.9 min. The temperature of the flask was 70°C. From the reactor the reaction mixture was flowing to a separator where the ingoing phases were separated. A gas chromatography analysis of the organic phase showed that the yield of the monoester (2 isomers) was 20.7 % and trimethylpentandiol 0.2 %

### Example 8

The same apparatus for continuous production as used in Ex. 7 was used with the difference that the shape of the reactor was somewhat different. Thus the reactor consisted of a glass tube having a diameter of 2.2 cm and a height of 18 cm. It was provided with an inlet and an outlet tube, a thermometer, an efficient reflux cooler, and a tube led through the reflux cooler, which tube reached the bottom of the reactor. The inner diameter of the latter tube was 2 mm. A stream of nitrogen gas was continuously fed through this tube and in this way a stirring of the reaction mixture was obtained. The pumps added isobutanal containing 1 % by weight of polyethylenglycol (M_{w} 400) and a 25 % solution of NaOH. The amount of NaOH, as 100%, was equal to 2.5% by weight of the amount of isobutanal added. The reactants were added to the reactor without being previously preheated. The dwell time of the reaction mixture in the reactor was 42 s. The reaction was initiated by heating the reactor to about 60°C. When the reaction started the heating bath was removed. The temperature of the reactor was stabilized at 69 to 70°C. The phases were separated in the separator. When the process had become stable samples were taken out for analysis. A gas chromatographic analysis showed that the yield of the monoester (2 isomers) was 30% calculated on the amount of isobutanal added. The corresponding yield of trimethyl pentadiol was 1%.

As a comparison it can be pointed out that when using a back-mixing process without phase transfer catalyst using NaOH solution only (US-A-3,718,689, Ex. 2) the dwell time in the reactor was 1 hr, i.e. 30 to 90 times longer than in a process according to the present invention.

## Claims

1. Process for the preparation of diols of the general formulae I 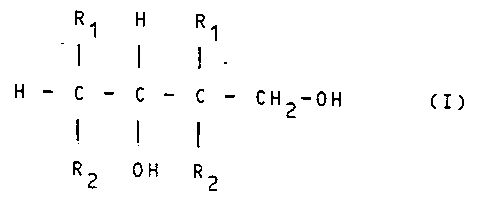 and/or its monoesters of the general formulae II 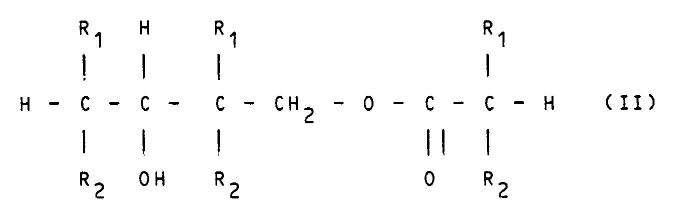 and/or the general formulae III 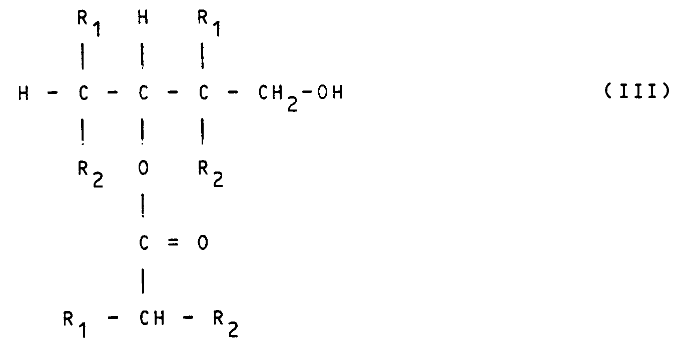 wherein R₁ and R₂ are the same or different and each denotes a lower alkyl having 1 to 4 carbon atoms, preferably a methyl, **characterized** in that aldehydes containing a hydrogen atom in alfa position are condensed in a multi phase reaction environment and having the general formulae IV 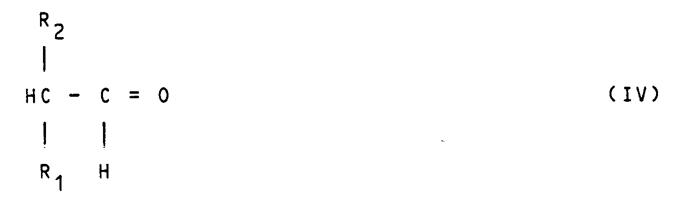 wherein R₁ and R₂ have the meanings given above, in the presence of a catalytic system consisting of a phase transfer catalyst and an alkaline substance.

2. Process according to claim 1, **characterized** in that the phase transfer catalyst consists of a quaternary ammonium compound, quaternary phosphonium compound, quaternary arsonium compound, a polyalkylene glycolether, and/or a cyclic polyether.

3. Process according to claim 1, **characterized** in that the aldehyde is isobutanal.

4. Process according to claim 1, **characterized** in that the molar ratio between base and aldehyde is 0.2 to 1.1:1 for the production of monoesters according to formulae II and/or III.

5. Process according to claim 1, **characterized** in that the molar ratio between the base and aldehyde is 0.5 to 2.0:1 for the production of a diol of the general formulae I.

6. Process according to claim 1, **characterized** in that the reaction is carried out at a temperature of 40 to 150°C, preferably 60 to 90°C.

7. Process according to one or more of claims 1 to 5, **characterized** in that the reaction is stopped by stopping an ongoing stirring and separation of the phases present, and/or by treating the alkaline compound present with an acid to neutralize the same, and/or by cooling the reaction mixture.

8. Process according to one or more of claims 1 to 7, **characterized** in that distillation of light reaction products and decomposition of polymeric structures formed by the aldehyde present take place in the presence of the phase transfer catalyst after finalized reaction.

9. Process according to claims 1 to 8, **characterized** in that the reaction is carried out batchwise.

10. Process according to claims 1 to 8, **characterized** in that the reaction is carried out continuously.

## Patentansprüche

1. Verfahren zur Herstellung von Diolen der allgemeinen Formel I 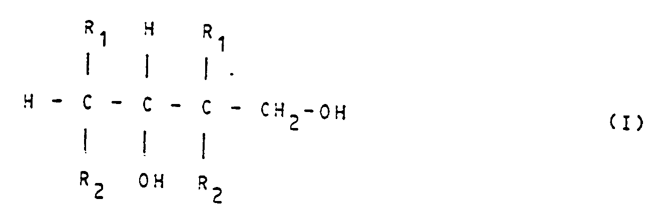 und/oder ihrer Monoester der allgemeinen Formel II 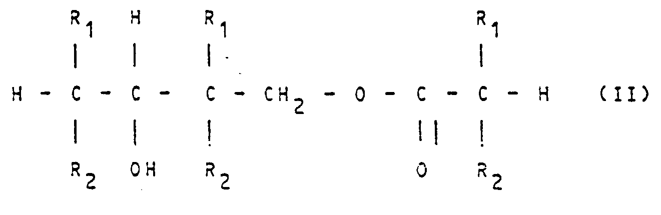 und/oder der allgemeinen Formel III 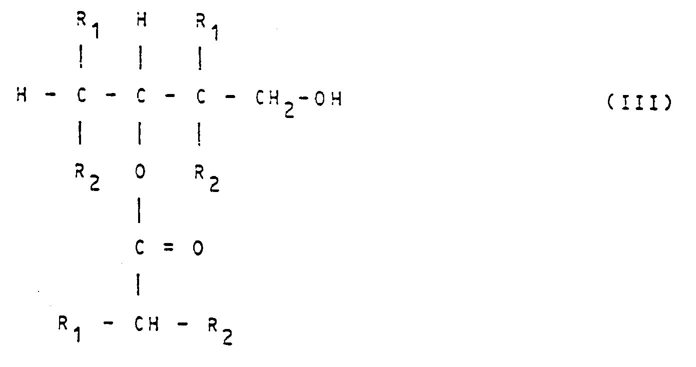 worin R₁ und R₂ gleich oder verschieden sind und jeweils eine niedermolekulare Alkylgruppe mit 1 bis 4 Kohlenstoffatomen, vorzugsweiseeine Methylgruppe, bedeuten, **dadurch gekennzeichnet**, daß Aldehyde, die ein Wasserstoffatom in alpha-Stellung enthalten und die allgemeine Formel IV 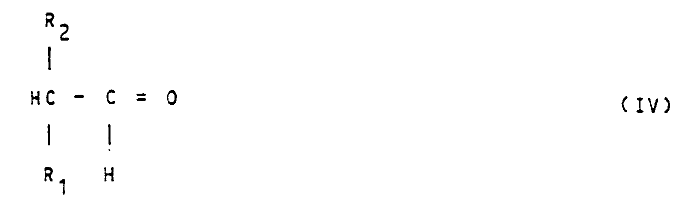 haben, worin R₁ und R₂ die obige Bedeutung besitzen, in einer mehrphasigen Reaktionsumgebung in Gegenwart eines katalytischen Systems kondensiert werden, das aus einem Phasenüberführungskatalysator und einer alkalischen Substanz besteht.

2. Verfahren nach Anspruch 1, **dadurch gekennzeichnet**, daß der Phasenüberführungskatalysator aus einer quaternären Ammoniumverbindung, einer quaternären Phosphoniumverbindung, einer quaternären Arsoniumverbindung, einem Polyalkylenglycolether und/oder einem zyklischen Polyether besteht.

3. Verfahren nach Anspruch 1, **dadurch gekennzeichnet**, daß der Aldehyd Isobutanal ist.

4. Verfahren nach Anspruch 1, **dadurch gekennzeichnet**, daß das Molverhältnis zwischen Base und Aldehyd 0,2 bis 1,1:1 für die Gewinnung von Monoestern gemäß den Formeln II und/oder III ist.

5. Verfahren nach Anspruch 1, **dadurch gekennzeichnet**, daß das Molverhältnis zwischen der Base und dem Aldehyd 0,5 bis 2,0:1 für die Gewinnung eines Diols der allgemeinen Formel I ist.

6. Verfahren nach Anspruch 1, **dadurch gekennzeichnet**, daß die Reaktion bei einer Temperatur von 40 bis 150 °C, vorzugsweise von 60 bis 90 °C durchgeführt wird.

7. Verfahren nach einem der oder mehreren der Ansprüche 1 bis 5, **dadurch gekennzeichnet**, daß die Reaktion angehalten wird, indem das weitere Rühren unterbrochen wird und die vorhandenen Phasen getrennt werden und/oder die vorhandene alkalische Verbindung mit einer Säure behandelt wird, um sie zu neutralisieren, und/oder das Reaktionsgemisch gekühlt wird.

8. Verfahren nach einem oder mehreren der Ansprüche 1 bis 7, **dadurch gekennzeichnet**, daß Destillation leichter Reaktionsprodukte und Zersetzung von durch den vorhandenen Aldehyd gebildeten polymeren Strukturen in Gegenwart des Phasenüberführungskatalysators nach beendeter Reaktion stattfinden.

9. Verfahren nach Anspruch 1 bis 8, **dadurch gekennzeichnet**, daß die Reaktion ansatzweise durchgeführt wird.

10. Verfahren nach Anspruch 1 bis 8, **dadurch gekennzeichnet**, daß die Reaktion kontinuierlich durchgeführt wird.

## Revendications

1. Procédé pour la préparation de diols de la formule générale : 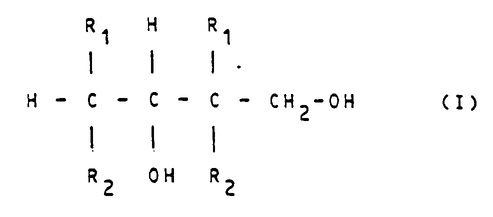 et/ou de leurs monoesters de la formule générale II : 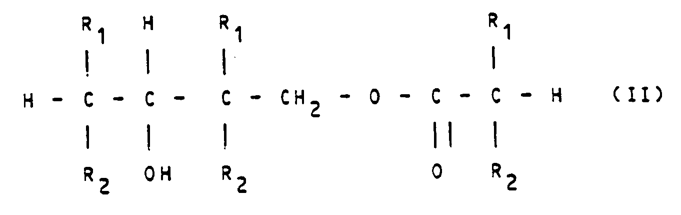 et/ou de la formule générale III : 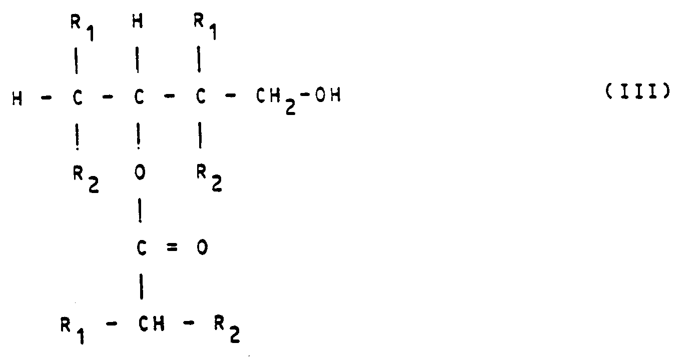 dans lesquelles R₁ et R₂ sont identiques ou différents et représentent chacun un groupe alkyle inférieur comportant 1 à 4 atomes de carbone, de préférence un groupe méthyle, caractérisé en ce que l'on condense des aldéhydes contenant un atome d'hydrogène en position alpha dans un environnement réactionnel à plusieurs phases et répondant à la formule générale IV : 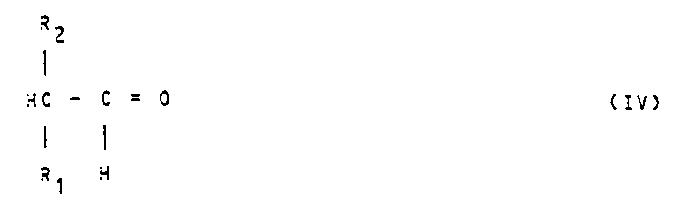 dans laquelle R₁ et R₂ ont les significations données précédemment, en présence d'un système catalytique formé d'un catalyseur de transfert de phase et d'une substance alcaline.

2. Procédé suivant la revendication 1, caractérisé en ce que le catalyseur de transfert de phase est un composé d'ammonium quaternaire, un composé de phosphonium quaternaire, un composé d'arsonium quaternaire, un polyalkylène glycoléther et/ou un polyéther cyclique.

3. Procédé suivant la revendication 1, caractérisé en ce que l'aldéhyde est l'isobutanal.

4. Procédé suivant la revendication 1, caractérisé en ce que le rapport molaire entre la base et l'aldéhyde est de 0,2 à 1,1/1 pour la production de monoesters des formules II et/ou III.

5. Procédé suivant la revendication 1, caractérisé en ce que le rapport molaire entre la base et l'aldéhyde est de 0,5 à 2,0/1 pour la production d'un diol de la formule générale I.

6. Procédé suivant la revendication 1, caractérisé en ce que la réaction est réalisée à une température de 40 à 150°C, de préférence de 60 à 90°C.

7. Procédé suivant l'une quelconque des revendications 1 à 5, caractérisé en ce que la réaction est arrêtée en arrêtant l'agitation en cours et par la séparation des phases présentes et/ou en traitant le composé alcalin présent avec un acide pour neutraliser celui-ci et/ou en refroidissant le mélange de réaction.

8. Procédé suivant l'une quelconque des revendications 1 à 7, caractérisé en ce que la distillation des produits de réaction légers et la décomposition des structures polymériques formées par l'aldéhyde présent se font en présence du catalyseur de transfert de phase après la fin de la réaction.

9. Procédé suivant l'une quelconque des revendications 1 à 8, caractérisé en ce que la réaction est réalisée d'une façon discontinue.

10. Procédé suivant l'une quelconque des revendications 1 à 8, caractérisé en ce que la réaction est réalisée de façon continue.
